# EUROPEAN PATENT SPECIFICATION

(11) **EP 2 245 197 B1**
(45) Date of publication and mention of the grant of the patent: **12.10.2016**
(21) Application number: 09707399.3
(22) Date of filing: 09.02.2009
(51) Int. Cl.: C12Q 1/68

(54) **IMPROVEMENTS IN AND RELATING TO ANALYSIS**
VERBESSERUNGEN BEI UND IN VERBINDUNG MIT DER ANALYSE
AMÉLIORATIONS APPORTÉES À L'ANALYSE

(30) Priority: 07.02.2008 US 26869 P
(43) Date of publication of application: 03.11.2010
(73) Proprietor: Whitespace Enterprise Corporation, Fountain Hills, AZ 85268 (US)
(72) Inventor: LENIGK, Ralf, Tempe, AZ 85281 (US); HURTH, Cedric, Michael, Tempe, AZ 85281 (US); NORDQUIST, Alan, Richard, Tempe, AZ 85281 (US); ZENHAUSERN, Frederic, Tempe, AZ 85281 (US); TULLY, Gillian, Solihull B37 7YN (GB); HOPWOOD, Andrew, John, Solihull B37 7YN (GB); KOUMI, Pieris, Solihull B37 7YN (GB)
(74) Representative: Pawlyn, Anthony Neil
(86) International application number: PCT/GB2009/000354
(87) International publication number: WO 2009/098485

(56) References cited:
- WO-A-2006/059132
- US-A1- 2004 034 211
- US-B1- 6 225 067
- US-B1- 7 081 226

## Description

### BACKGROUND OF THE INVENTION

This invention concerns improvements in and relating to analysis, and in particular, but not exclusively, apparatus and methods for parallel analysis of a sample.

In a wide variety of situations it is desirable to be able to quickly and accurately analyse samples, particularly, biological samples. Such samples may be being considered in a medical context, for instance the diagnosis of a disease or medical condition, or in a forensic science context, for instance the determination of a DNA profile from a sample.

There is also an increasing drive towards miniaturisation of apparatus and methods for considering such samples. This is with a view to making the apparatus and methods more portable and easy to use at the optimum location and with a view to minimising the size of sample required for accurate and complete analysis. To gain the full benefits of miniaturisation, the apparatus and method must perform the analysis quickly.

Prior art approaches have involved methods in which the main analysis method is performed with the benefit of information obtained from a subsidiary analysis method. This approach may improve the accuracy of the main analysis method, but represents a time delay which must be incurred before the main analysis method can be started. As a result, the completion of the main analysis method is also delayed. In the context of miniaturised methods and apparatus, the conduct of the subsidiary analysis method also introduces complications to sample handling and storage in respect of that part of the sample to be used in the main analysis method.

US7081226 provides a thermal cycling method and apparatus in which feedback from the results of early cycles in the processing of a sample is used for control and optimisation of the process with respect to the later cycles applied to the same sample.

US2204/0034211 and WO2006/059132 provide analysis processes in which the results from one or more analyses are considered after the end of a process and are used to modify the process in one or more further analyses, with those one or more further analyses only starting after the one or more analyses have finished.

### SUMMARY OF THE INVENTION

The present invention seeks to address the problems identified and other problems by providing for the parallel performance of the subsidiary analysis method and the main analysis method. The results of the subsidiary analysis method are obtained, considered and potentially used to amend the main analysis method before it is completed. In this way, the main analysis method is performed as accurately as possible, but with no time delay due to the subsidiary analysis method also being conducted. Analysis time is minimised, whilst the method and apparatus provide for optimal characterisation, classification, diagnosis or analysis. Additionally, the sample to be considered can be split into two parts, one going to the subsidiary analysis method for consideration and the other going to the main analysis method for consideration at the same time. As a result, the process for collecting, splitting and using the sample is simplified and there are no added complexities from storing or handling the sample for use in the main analysis method, prior to its use.

According to a first aspect of the invention we provide a method of analysing a sample, the method including:
providing a first part of the sample;
providing a second part of the sample;
the first part of the sample and the second part of the sample being separate from each other;
conducting a first analysis on the first part of the sample using a first analysis data processor;
considering the results of the first analysis;
conducting a second analysis on the second part of the sample, the first analysis and the second analysis being different analysis processes, the second analysis being conducted according to a procedure or a further procedure, the procedure using a value for each of one or more characteristics of the procedure, the further procedure using a different value for one or more of the characteristics;
wherein the consideration of the results of the first analysis is used to determine whether the procedure or a further procedure is used, the procedure being provided for use when the signals received from the first analysis data processor are of a given form, the further procedure being provided for use when the signals received from the first analysis data processor differ from the given form; and
wherein the second analysis is started before the results of the first analysis are obtained.

According to a second aspect of the invention we provide apparatus for analysing a sample, the including:
a sample introduction location;
a first chamber in fluid communication with the sample introduction location;
a first chamber process controller;
a first detector for one or more properties of a first part of a sample processed in the first chamber according to the first chamber process controller;
a first analysis data processor receiving signals from the first detector and providing signals to a second process controller;
a second chamber in fluid communication with the sample introduction location, the second chamber processing a second part of the sample according to the second process controller;
a second detector for one or more properties of the second part of the sample processed in the second chamber;
the first part of the sample in the first chamber and the second part of the sample in the second chamber being separate from each other;
the first analysis and the second analysis being different analysis processes;
the second process controller providing a procedure for application to the second chamber, the procedure using a value for each of one or more characteristics of the procedure, the procedure being provided for use when the signals received from the first analysis data processor are of a given form, the second process controller providing a further procedure for application to the second chamber, the further procedure using a different value for one or more of the characteristics, the further procedure being applied to the second chamber in response to the signals provided by the first data processor to the second process controller when the signals differ from the given form, and
wherein the signals from the first analysis data processor are provided to the second process controller after the second process controller has started applying the procedure to the second chamber.

According to a third aspect of the invention we provide apparatus for analysing a sample, the including:
a sample introduction location;
a first chamber in fluid communication with the sample introduction location;
a first chamber process controller;
a first detector for one or more properties of a first part of a sample processed in the first chamber according to the first chamber process controller;
a first analysis data processor receiving signals from the first detector and providing signals to a second analysis data processor;
a second chamber in fluid communication with the sample introduction location;
a second chamber process controller;
a second detector for one or more properties of the second part of the sample processed in the second chamber according to the second chamber process controller;
the first part of the sample in the first chamber and the second part of the sample in the second chamber being separate from each other;
the first analysis and the second analysis being different analysis processes;
the second analysis data processor providing a procedure for application to signals received from the second detector, the procedure using a value for each of one or more characteristics of the procedure, the procedure being provided for use when the signals received from the first analysis data processor are of a given form, the second analysis data processor providing a further procedure for application to the signals from the second detector, the further procedure using a different value for one or more of the characteristics, the further procedure being applied to the signals from the second detector to the second process controller when the signals differ from the given form, and
wherein the signals from the first analysis data processor are provided to the second analysis data processor after the second process controller has started applying the process to the second chamber.

The first and/or second and/or third aspects of the invention may include any of the features, options or possibilities provided for in this document, including from amongst the following.

The sample may be a biological sample. The sample may be a sample of nucleic acid. The sample may be a sample of DNA. The sample may be of animal or plant or bacterial origin.

The sample may be collected from a person. The sample may be collected from a location, other than on or in a person. The sample may be a blood sample or bodily fluid sample or sample containing cells or parts thereof.

The sample may be processed before providing the first part and the second part from the sample. The sample may be processed by the addition of one or more chemical species. The sample may be processed to provide the nucleic acid or DNA in a form adapted to amplification. The sample may be processed by being diluted. The sample may be processed by being purified.

The sample may be formed into only a first part and a second part. The sample may be formed into a first part, a second part and one or more further parts. The one or more further parts may be analysed or may be discarded. Preferably the first part and the second part have the same volume +/- 10%, more preferably +/- 5% and ideally +/-1%. Preferably the first part and the second part have the same volume.

Preferably the first art and second part have the same volume and the same volume is used in two or more uses of the method of analysis.

One or more reagents may be added to the sample or to the first part and the second part. The same reagents may be added to each part, but preferably the reagents added to the first part and to the second part are different. One or both of the reagents may include primers, and preferably a multiplex or multimix. Preferably the ratio of sample to reagent(s) is the same in respect of the first part and in respect of the second part +/- 10%, more preferably +/- 5% and ideally +/-1%. The ratio of sample to reagent(s) may be the same.

Preferably, the first part of the sample is separated from the second part of the sample. Preferably the first part is fed to a separate chamber to the second part. Preferably the first analysis of the first part is performed in a separate chamber to the second analysis of the second part.

The first analysis may be a subsidiary analysis. The first analysis may be provided in a subsidiary analysis stage. The first analysis may include first analysis data processing, for instance provided by a first analysis data processing stage.

The first analysis may provide information on one or more characteristics of the first part of the sample. The one or more characteristics are preferably characteristics which affect the second analysis, particularly one or more of: the speed of the second analysis, the accuracy of the second analysis, the reliability of the second analysis. The one or more characteristics may include one or more of: the quantity of nucleic acid, the quantity of amplifiable nucleic acid, the quantity of amplifiable nucleic acid of a given type, the presence of one or more inhibitors to amplification, the extent of degradation of the nucleic acid, the presence of Y chromosome nucleic acid, the quantity of Y chromosome nucleic acid, the presence of nucleic acid from two or more different sources; the ratio of the nucleic acid from one source to the nucleic acid from another source. The first analysis, potentially together with the first analysis data processing, may provide the information on the one or more characteristics.

The first analysis data processing may be provided by a computer implemented method or data processing unit. The first analysis data processing may be applied to the analysis results from the first analysis. The first analysis data processing may provide interpretation of the first analysis results.

The considering of the results of the first analysis may provide feedback to the second analysis. Preferably the feedback determines whether the value for one or more of the characteristics of the procedure is changed to a different value in the second analysis. The feedback may be provided automatically to the second analysis. The feedback may be reviewed by a user before being used in the second analysis. The feedback may result in the second analysis being unchanged.

The second analysis may be a main analysis. The second analysis may be provided in a main analysis stage. The second analysis may include second analysis data processing, for instance provided by a second analysis data processing stage.

The second analysis data processing may be provided by a computer implemented method or data processing unit. The second analysis data processing may be applied to the analysis results from the second analysis. The second analysis data processing may provide interpretation of the second analysis results.

The second analysis may provide information on the sample. The information may be the presence or absence of one or more features of or in the sample. The information may be a nucleic acid profile for the sample. The information may be a genotype for the sample. The second analysis may be conducted according to a procedure which has a standard form. The standard form of the procedure may be used in the second analysis unless the consideration of the results from the first analysis suggest a change to the procedure. The procedure may be defined in terms of one or more characteristics, the characteristics including one or more of: a number of PCR cycles to be applied to the second part of the sample; a total length of time for a given PCR cycle, in respect of one or more or all of the PCR cycles; a length of time for a denaturing part of a given PCR cycle, in respect of one or more or all of the PCR cycles; a length of time for an annealing part of a given PCR cycle, in respect of one or more or all of the PCR cycles; a length of time for an extension part of a given PCR cycle, in respect of one or more or all of the PCR cycles; an activation temperature for PCR; a denaturing temperature for a given PCR cycle, in respect of one or more or all of the PCR cycles; an annealing temperature for a given PCR cycle, in respect of one or more or all of the PCR cycles; an extension temperature for a given PCR cycle, in respect of one or more or all of the PCR cycles; the quantity of one or more reagents to add during the reaction. The value for a characteristic is preferably a number of cycles or a temperature or a length of time.

The value for one or more of the characteristics may be changed in response to the first analysis indicating one or more features for the sample. The one or more features may include the presence of one or more inhibitors in the sample. Where one or more inhibitors are detected, the value of the number of PCR cycles may be increased. The one or more features may include the quantity of nucleic acid in the sample or first part thereof. The quantity of nucleic acid may be compared with a reference quantity or reference range of quantities, the reference quantity or range of quantities being associated with one or more values to use in the second analysis. Where the quantity detected in the first analysis is below the reference quantity or reference range of quantities, the number of cycles may be increased compared with the value of cycles associated with the references. Where the quantity detected in the first analysis is above the reference quantity or reference range of quantities, the number of cycles may be decreased compared with the value of cycles associated with the references. A comparison and adjustment of this type may be made with respect to a series of different reference values and/or ranges of reference values. The quantity of nucleic acid determined in the first analysis may be used to define the values for one or more of the characteristics, particularly the number of cycles, used in the second analysis to achieve a desired concentration or amount of amplified nucleic acid.

The method may use the feedback from the first analysis, directly or via the first analysis data processing, to the procedure for the second analysis to optimise one or more features of the second analysis, for instance the performance level and/or level of control of the amplification in the second analysis. The method may use the feedback from the first analysis, directly or via the first analysis data processing, to the procedure for the second analysis to reduce the second analysis time for completion compared with the unaltered values for the one or more characteristics of the procedure and/or to provided reduced reagent consumption compared with the unaltered values for the characteristics of the procedure and/or to provide a more accurate quantification of the nucleic acid compared with the unaltered values for the one or more characteristics of the procedure.

The consideration of the results of the first analysis used to determine whether the value for one or more of the characteristics of the procedure is changed to a different value, may be in respect of a value used in the second analysis in the physical processing of the second part of the sample or in the second analysis data processing.

The feedback from the first analysis to the second analysis may be provided to the physical processing of the second part of the sample and/or the second analysis data processing.

When applied to the second analysis data processing in particular, the feedback from the first analysis may indicate whether or not the sample is from a mixture of sources and/or the ratio of nucleic acid from one source to that from another source in the mixture. The value for a characteristic which may be changed in the second analysis is whether or not the sample is a mixture and/or the ratio of one source of nucleic acid to another source. The value may be the presence or absence of heterozygous balance, for instance within a given range. The value may be the presence or absence of allele drop out.

The first analysis and the second analysis may start at the same time. Where the first analysis and the second analysis involve a PCR based reaction, that reaction may be started at the same time. The same time may be precisely the same time. The same time may be within 5 minutes of the other analysis starting, preferably within 3 minutes of the other analysis starting, more preferably within 1 minute of the other analysis starting and ideally within 20 seconds of the other analysis starting.

The first analysis may take less than 90 minutes to complete, preferably less than 70 minutes to complete, more preferably less than 60 minutes to complete, still more preferably less than 50 minutes to complete and ideally 45 minutes or less to complete. The first analysis may take at least 60 minutes to complete, potentially at least 40 minutes to complete and preferably at least 20 minutes to complete. The completion of the first analysis may include the time required for the first analysis data processing to be completed. The completion of the first analysis may exclude the time required for the first analysis data processing to be completed.

The second analysis may have a scheduled completion time, for instance, according to the values for each of one or more characteristics of the procedure before any variation to such values. The first analysis may be completed at least 10 minutes before the scheduled completion time of the second analysis, preferably at least 20 minutes before, more preferably at least 30 minutes before and ideally at least 40 minutes before. The first analysis may be started at a time so as to be completed at least a given time in advance of the scheduled completion of the second analysis.

The second analysis may take less than 150 minutes to complete, preferably less than 120 minutes to complete, more preferably less than 105 minutes to complete, still more preferably less than 90 minutes to complete. The second analysis may take at least 60 minutes to complete, potentially at least 75 minutes to complete and preferably at least 90 minutes to complete. The completion of the second analysis may include the time required for the second analysis data processing to be completed. The completion of the second analysis may exclude the time required for the second analysis data processing to be completed.

The first analysis and the second analysis may both be in operation at the same time for at least 10 minutes, more preferably at least 20 minutes and ideally at least 30 minutes. The first analysis and the second analysis may both involve the same type of reaction, for instance a PCR based reaction. The first analysis and second analysis may involve different analysis processes. The different analysis processes may differ in terms of one or more of: the reaction involved, the reagents involved, one or more characteristics of the part being analysed.

The sample introduction location may be a chamber. The first chamber and/or the second chamber may be in fluid communication with a further chamber provided with the first detector and/or second detector.

A first detector may be provided for one or more properties of the first part of the sample processed in the first chamber according to the first chamber process controller. A second detector may be provided for one or more properties of the second part of the sample processed in the second chamber according to the second chamber controller. The first detector and the second detector may be one and the same detector. At one time the detector may serve as the first detector and at another time the detector may serve as the second detector. The detector and/or first detector and/or second detector may analyse the first part of the sample and/or the same part of the sample in different chambers or in the same chamber at different times.

The first chamber process controller may be a part of a unitary process controller. The second chamber process controller may be a part of a unitary process controller. The unitary process controller may provide the same and/or different process control to the first chamber and the second chamber.

The first and/or second detector may be incorporated into a wall of a chamber. The first and/or second detector may detect fluorescence excitation and/or emitted fluorescence. The first and/or second detector may be connected to the chamber by an optical fibre. The chamber may be provided with a source of excitation, for instance a laser. The apparatus may provide an angle of about 90 degrees between the excitation source and the collection of emitted light. The first and/or second detector may provide an optical detection. The first and/or second detector may be a charge-coupled device. The first and/or second detector may include a spectrometer, for instance for a fluorescent signal emitted by one or more dyes chemically linked to the biological sample to be analysed. An emission filter may be placed between the first and/or second detector and the light source, for instance, as a filter provided in front of a spectrometer device, potentially, to minimize the influence of stray scattered excitation light, and an excitation light generated preferably by a compact LED-based source:

### DESCRIPTION OF THE DRAWINGS

Various embodiments of the invention will now be described, by way of example only, and with reference to the accompanying drawings in which:
Figure 1 is a schematic illustration of the parallel reactions and feedback process of an embodiment of the invention;
Figure 2 is an example of a chamber for real-time PCR and optical detection utilizing optical fibres; and
Figure 3 is an illustration of one coupling possibility combining a real-time PCR bioreactor and optical fibre-based detection on a single fluidic cartridge.

### DETAILED DESCRIPTION OF THE PREFERRED EMBODIMENTS

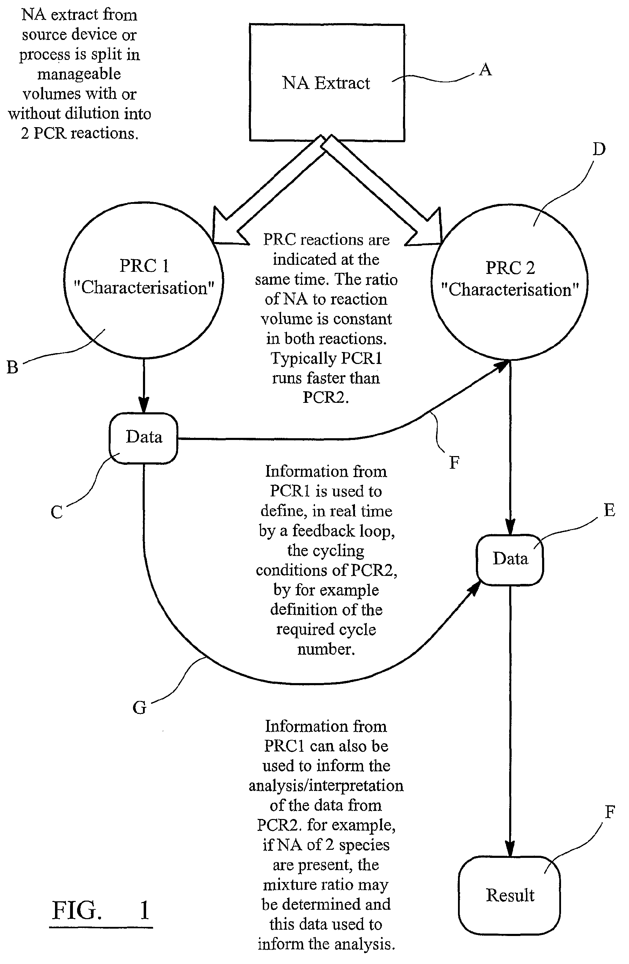

As illustrated schematically in Figure 1, the method involves an extraction stage, A, subsidiary analysis stage, B, subsidiary analysis data processing stage, C, main analysis stage, D, main analysis data processing stage, E, and result presentation stage F.

The extraction stage, A, involves the collection of a nucleic acid sample and its preparation to a form suitable for analysis.

The sample could be from a blood sample, bodily fluid sample, cells or other biological sample. The sample could be from an environmental source. The sample could be taken directly from a person, for instance using a swab or syringe, or the sample could be collected indirectly, for instance from a surface at a crime scene. The extraction stage, A, may include any of the steps necessary to place the nucleic acid in a form suitable for analysis. This could include dilution, cell disruption, buffering, addition of reagents or the like.

Once the extraction stage, A, is completed, the sample is split into two parts. This means that the ratio of sample to reaction mix is identical in each of the two reactions to ensure the sample behaves in the same manner in each, whether or not affected by concentration or inhibition effects. In other situations, the sample may be split into a different number of parts, for instance three parts, according to the processing or analysis requirements. The dimensions and/or cross-sections and/or surface properties of the channels provided can be configured to ensure that the split is achieved into the desired number of parts and in the desired proportions for each.

The first part, a subsidiary analysis part, is fed to the subsidiary analysis stage, B. The second part, a main analysis part, is fed to the main analysis stage, D. The subsidiary analysis stage, B, and main analysis stage, D, are conducted in separate reaction chambers.

Whilst the reagents and other materials necessary for the analysis can be added before the sample is split into the parts, as an alternative, the reagents and other materials necessary for the analysis can be provided to the reaction chambers separately. In each case, the reagents may be supplied from one or more further chambers provided as a part of the apparatus performing the invention or they may be supplied externally. In either case, suitable channels are provided to convey the reagents to the sample or parts of the sample.

In the context of a nucleic acid sample, a PCR-based reaction is performed in each of the subsidiary analysis stage, B, and the main analysis stage, D. The processing of both stages commences at the same time. In this way, a sample containing nucleic acid is amplified simultaneously using an enzymatic-based amplification reaction, preferably a real-time PCR-based approach, in both the subsidiary, B, and main analysis, D, stages as quickly as possible. Details of the PCR-based reaction are available from many sources, including US4683195 and US4683 202.

The subsidiary analysis stage, B, together with the subsidiary analysis data processing stage, C, is intended to reveal key information about the nature, characteristics or properties of the sample which may be material to its efficient processing. For example, the performance of the main analysis stage, D, may be impacted upon by features of the sample, such as the quantity of amplifiable nucleic acid, the quantity of amplifiable nucleic acid of a given type, the presence of one or more inhibitors to amplification in the sample and the state of degradation of the nucleic acid in the sample. Information as to the quantity of Y chromosome nucleic acid present can also be useful. The subsidiary analysis stage, B, and subsidiary analysis processing stage, D, seek to inform on one or more of these in their results. As the results of the PCR-based reaction are only available through analysis, after the PCR-based reaction has been completed, no knowledge as to the extent of progress and success of the PCR-based reaction is apparent whilst it is in progress. PCR provides a product intended for analysis after PCR is completed. However, in the present invention, the results from the subsidiary analysis stage, B, can be obtained before the main analysis stage, D, is completed.

The time required to complete the subsidiary analysis stage, B, and process its results is less than the time taken to complete the main analysis stage, D. Process times of between 20 and 45 minutes are typical for the subsidiary analysis stage, B, and subsidiary analysis data processing stage, C. As a result, the results from the subsidiary analysis are available whilst the main analysis stage, D, is still in progress. Main analysis stage, D, process times of 60 to 120 minutes are typical. As a result, the main analysis stage, D, may be only 1/3^{rd} of the way through when the results for the subsidiary analysis stage, B, become available. Depending upon the results obtained from the subsidiary analysis, changes may be applied to the main analysis stage, D. A variety of possible changes exist and are discussed in more detail below.

The subsidiary analysis stage, B, may provide a series of PCR cycles which are of shorter duration than the main analysis stage, D, so that certain characteristics of the sample can be determined. These characteristics may include the extent of amplification achieved in the subsidiary analysis stage. More detailed options would include quantification of the amount of autozomal DNA detected and/or amount of Y chromosome DNA detected and/or extent of inhibition of PCR observed.

In a PCR-based reaction, a plot of fluorescence (a measure of nucleic acid quantity) against number of cycles completed (a measure of time) provides a plot having a linear mid section. The subsidiary analysis stage, B, is intended to give a fluorescence value within the linear part of the plot having an anticipated value or an anticipated value range. If the observed value is less than the anticipated value or below the anticipated value range, then less amplification than intended has been achieved and the extent of amplification in the main analysis stage, D, can be increased, for instance, by increasing the number of cycles. If the observed value is greater than the anticipated value or anticipated range of values, then the extent of amplification in the main analysis stage, D, may be decreased, for instance by decreasing the number of cycles.

Whilst direct feed back from the subsidiary analysis stage, B, to the main analysis stage, D, is possible, the results of the subsidiary analysis stage, B, will generally be processed in the subsidiary analysis data processing stage, C, first. Thus feedback, F, is provided.

The subsidiary analysis data processing stage, C, comprises a computer implemented data processing unit which receives the results of the analysis provided by the subsidiary analysis stage, B, and processes those to generate additional information. The subsidiary analysis data processing stage, C, may interpret the results to provide the additional information, for instance according to one or more pre-determined criteria or sets of criteria. The feedback, F, can be provided automatically, or with user intervention or review.

The provision of feed back from the subsidiary analysis stage B, directly, or via subsidiary analysis data processing stage, C, to the main analysis stage, D, optimises performance and provides a more controlled amplification in the main analysis stage, D. A preferred outcome of the invention, is a more controlled amplification in the main analysis stage, D, and the optimization of the reaction(s) conditions for better quantification of the reaction products. This could lead to faster cycle times, lower reagent(s) consumption, cost reduction and more accurate quantification of nucleic acids and some of their reaction byproducts, by the main analysis stage, D.

A wide variety of possible changes to the main analysis stage, D, are possible.

For instance, if the presence of inhibitors is detected in the subsidiary analysis stage, and hence likely inhibition of nucleic acid in the main analysis stage, the number of cycles of amplification could be increased. As the quantity of nucleic acid resulting from amplification is related to the amplification efficiency of a cycle multiplied by the number of cycles, the presence of inhibitors (which will decrease the amplification efficiency of a cycle) can be overcome by increasing the number of such cycles, so as to get an equivalent quantity of nucleic acid after amplification has been completed.

For example, if the subsidiary analysis stage provides for the quantification of the amount of nucleic acid present in the sample, the quantity determined can be used to set the number of cycles of amplification necessary to achieve the desired concentration or amount of amplified nucleic acid. Where the quantity is low, the number of cycles could be increased; where the quantity is high, the number of cycles could be decreased. For example, the indication of a 1:1 mixture (e.g. the sample contains both male and female DNA or two species of DNA of interest) might result in the addition of a single cycle of PCR, for instance, to ensure optimal peak heights are achieved upon electrophoresis of the sample. Other variables would include the temperatures used for one or more parts of the PCR process, duration of one or more of the parts of the PCR process.

The feedback from the subsidiary analysis stage, B, can be provided by using detector(s) to analyse the subsidiary analysis stage, B, amplification products and so generate analysis signals. These can be processed and used to generate control signals sent to the apparatus controlling the PCR-based reaction in the main analysis stage, D. The control signals could be used direct or further processed to influence the PCR-based reaction.

As well as providing for feedback, F, from the subsidiary analysis to the main analysis stage, D, an alternative or additional feedback route, G, can be used.

After the main analysis stage, D, has generated its results, these are processed in a main analysis data processing stage E. The main analysis data processing stage, E, also comprises a computer implemented data processing unit which, in this case, receives the results of the analysis provided by the main analysis stage, D, and processes those to generate additional information. The main analysis data processing stage, E, may interpret the results to provide the additional information, for instance according to one or more pre-determined criteria or sets of criteria. The i³ nucleic acid interpretation software provided by Forensic Science Service Limited is one suitable tool for use in the main analysis data processing stage, E. The feedback, G, can be provided automatically, or with user intervention or review.

Whilst the main analysis data processing stage, E, can act on the results received from the main analysis stage, D, without further input, further advantages can be obtained by providing feedback, G, to the main analysis data processing stage, E. This feedback, G, can influence the processing applied by main analysis data processing stage, E. Again a variety of possible changes to the conduct of the main analysis data processing stage, E, can be made to account for various different forms for the sample according to the information obtained. The aim again is to use the information to provide improved performance from the main analysis data processing stage, E.

Amongst the possible issues to take into account are the following. The subsidiary analysis might identify 2 different "types" of nucleic acid in the sample and this could be used to change the processing by the main analysis data processing stage, by causing the sample to be interpreted as a mixture and potentially as a mixture based on a known mixture ratio established by the subsidiary analysis. The Plexor qPCR assay available from Promega Corporation, 2800 Woods Hollow Road, Madison, Wisconsin, USA, and described in US6242235, enables the simultaneous determination of both total human and total male DNA. Strategies for interpretation of heterozygous balance and allele drop out, available for use by the main analysis data processing stage, E, might also be applied depending upon the feedback, G, from the subsidiary analysis.

Amongst the important additional advantages of the invention, is one with particular importance in the context of miniaturised systems, such as lab-on-chip devices. In previous approaches, the analysis tends to have been operated based upon the provision of a constant quantity of nucleic acid; instead a constant volume is used in the present invention. This greatly simplifies the sample collection and preparation parts of the process. In miniaturised systems, such as those using simple fluidic manipulation of a sample, a fixed volume can be dispensed to the reactions far more easily. The feedbacks, F, G, allow for the variations in nucleic acid quantity encountered to be accounted for by adjusting the conditions of the reaction in a manner modified to suit the sample make up. This is a marked contrast with having to identify the volume needed to give the desired quantity of nucleic acid and then having to accurately meter that small volumes to provide the nucleic acid quantity.

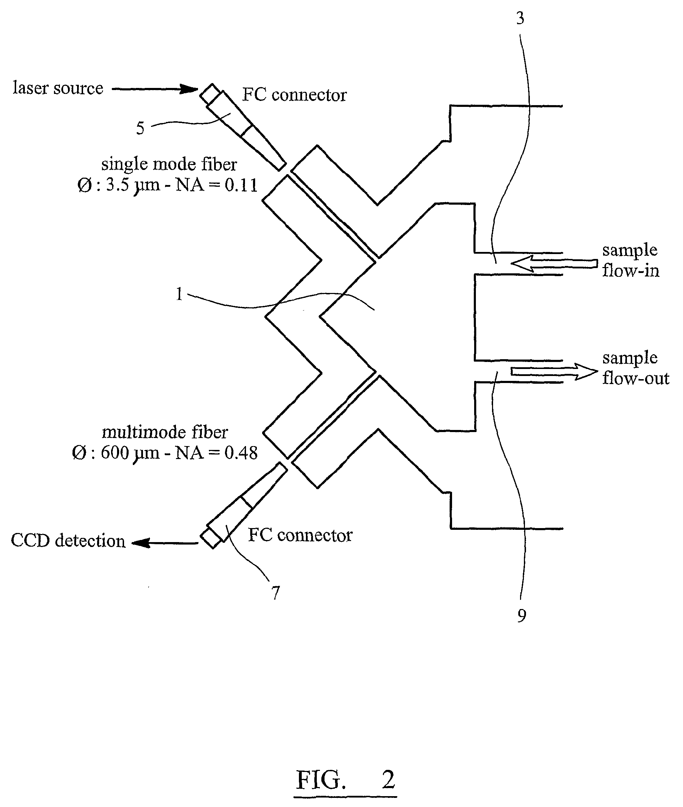

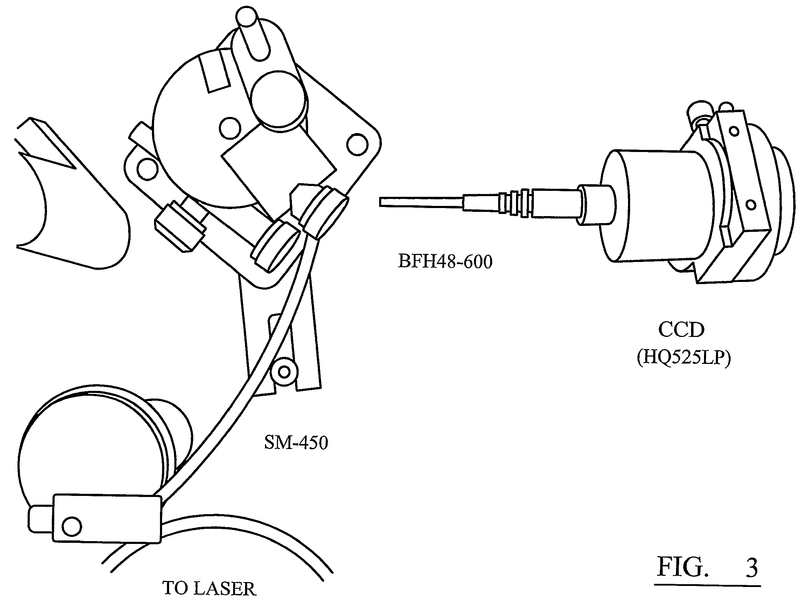

By way of example, in Figure 2, a chamber for use in extracting information from the reaction products of the subsidiary analysis stage, B, or main analysis stage, D, is shown. The reaction products, in the processed sample, are fed into the chamber 1, through an inlet 3. When inside the chamber 1, the reaction products are exposed to laser light from a laser source, not shown, with the light being conveyed to the chamber 1 along a single mode fibre optic 5. Other light sources, for instance, LED's can be used. This technique, laser induced fluorescence, LIF, uses the ability of dye molecules, associated with the amplified nucleic acid, to absorb light at one frequency and emit it at another frequency, to reveal the presence of the dye molecule and hence the nucleic acid, in a quantitative manner. A sample of the interaction of the light with the reaction products, the emitted fluorescence frequency and intensity, is obtained through multi-mode fibre 7, which in turn is connected to a CCD detector, not shown. This CCD detector converts the sample of the interaction of the light with the reaction product into electrical signals which can then be processed in the subsidiary analysis data processing stage, C, or main analysis data processing stage, E, as appropriate. Once the reaction products in the sample have been considered, they are removed from the chamber 1 through outlet 9. The chamber 1 can then be purged or cleaned, prior to reuse in considering another sample. A physical embodiment of such a system is shown in Figure 3.

## Claims

1. A method of analysing a sample [A], the method including:
providing a first part of the sample;
providing a second part of the sample;
the first part of the sample and the second part of the sample being separate from each other;
conducting a first analysis [B] on the first part of the sample using a first analysis data processor [C];
considering the results of the first analysis [B];
conducting a second analysis [D, E] on the second part of the sample, the first analysis [B] and the second analysis [D, E] being different analysis processes, the second analysis [D, E] being conducted according to a procedure or a further procedure, the procedure using a value for each of one or more characteristics of the procedure, the further procedure using a different value for one or more of the characteristics;
wherein the consideration [F, G] of the results of the first analysis [B] is used to determine whether the procedure or a further procedure is used, the procedure being provided for use when the signals received from the first analysis data processor are of a given form, the further procedure being provided for use when the signals received from the first analysis data processor differ from the given form; and
wherein the second analysis [D, E] is started before the results of the first analysis [B] are obtained.

2. The method of claim 1, wherein the first analysis [B] and the second analysis [D, E] start within 5 minutes of the other analysis starting.

3. The method of claim 1 or claim 2, wherein the first analysis [B] takes less than 60 minutes to complete.

4. The method of any preceding claim, wherein the first analysis [B] is completed at least 30 minutes before the scheduled completion of the second analysis [D, E].

5. The method of any preceding claim, wherein the first part and the second part have the same volume +/-1%.

6. The method of any preceding claim, wherein the ratio of sample to reagents is the same in respect of the first part and in respect of the second part +/-1%.

7. The method of any preceding claim, wherein the first analysis [B] provides information [C] on one or more characteristics of the first part of the sample, the one or more characteristics being one or more of:
the quantity of nucleic acid;
the quantity of amplifiable nucleic acid;
the quantity of amplifiable nucleic acid of a given type;
the presence of one or more inhibitors to amplification;
the extent of degradation of the nucleic acid;
the presence of Y chromosome nucleic acid;
the quantity of Y chromosome nucleic acid;
the presence of nucleic acid from two or more different sources;
the ratio of the nucleic acid from one source to the nucleic acid from another source.

8. The method of any preceding claim, wherein the second analysis [D, E] is conducted according to a procedure which has a standard form and the standard form of the procedure is used in the second analysis [D, E] unless the consideration [F, G] of the results from the first analysis [B] suggest a change to the procedure.

9. The method of any preceding claim, wherein the procedure is defined in terms of one or more characteristics, and the value for a characteristic is a number of PCR cycles or a PCR cycle operating temperature or a length of time for a PCR cycle.

10. The method of any preceding claim, wherein the value for one or more of the characteristics of the procedure is changed in response to the first analysis [B] indicating the presence of one or more inhibitors in the sample [A].

11. The method of any preceding claim, wherein the quantity of nucleic acid is detected in the first analysis [B] and is compared with a reference quantity or reference range of quantities, the reference quantity or range of quantities being associated with one or more values to use in the second analysis [D, E], where the quantity detected in the first analysis [B] is below the reference quantity or reference range of quantities, the number of cycles being increased compared with the value of cycles associated with the references and/or where the quantity detected in the first analysis [B] is above the reference quantity or reference range of quantities, the number of cycles being decreased compared with the value of cycles associated with the references.

12. The method of any preceding claim, wherein the consideration of the results of the first analysis [B] is used to determine whether the value for one or more of the characteristics of the procedure is changed to a different value and the value is in respect of a value used in the second analysis [D, E] in the physical processing [D] of the second part of the sample or in the second analysis [D, E] in the second analysis data processing [E] of the results for the second part of the sample.

13. The method of any preceding claim, wherein the results of the first analysis [B] indicate whether or not the sample is from a mixture of sources and/or the ratio of nucleic acid from one source to that from another source in the mixture.

14. A method of analysing a sample, the method including: processing in parallel a first analysis method [B] and a second analysis method [D, E], the results of the first analysis method [B] being considered [F, G] and used to amend the second analysis method [D, E] before it is completed.

15. Apparatus for analysing a sample [A], the apparatus including:
a sample introduction location;
a first chamber in fluid communication with the sample introduction location;
a first chamber process controller;
a first detector for one or more properties of a first part of a sample processed in the first chamber according to the first chamber process controller;
a first analysis data processor [C] receiving signals from the first detector and providing signals to a second process controller;
a second chamber in fluid communication with the sample introduction location, the second chamber processing a second part of the sample according to the second process controller;
a second detector for one or more properties of the second part of the sample processed in the second chamber;
the first part of the sample in the first chamber and the second part of the sample in the second chamber being separate from each other;
the first analysis [B] and the second analysis [D, E] being different analysis processes;
the second process controller providing a procedure for application to the second chamber, the procedure using a value for each of one or more characteristics of the procedure, the procedure being provided for use when the signals received from the first analysis data processor [C] are of a given form, the second process controller providing a further procedure for application to the second chamber, the further procedure using a different value for one or more of the characteristics, the further procedure being applied to the second chamber in response to the signals provided by the first data processor [C] to the second process controller when the signals differ from the given form, and
wherein the signals from the first analysis data processor [C] are provided to the second process controller after the second process controller has started applying the procedure to the second chamber.

16. Apparatus for analysing a sample [A], the apparatus including:
a sample introduction location;
a first chamber in fluid communication with the sample introduction location;
a first chamber process controller;
a first detector for one or more properties of a first part of a sample processed in the first chamber according to the first chamber process controller;
a first analysis data processor [C] receiving signals from the first detector and providing signals to a second analysis data processor;
a second chamber in fluid communication with the sample introduction location;
a second chamber process controller;
a second detector for one or more properties of the second part of the sample processed in the second chamber according to the second chamber process controller;
the first part of the sample in the first chamber and the second part of the sample in the second chamber being separate from each other;
the first analysis [B] and the second analysis [D, E] being different analysis processes;
the second analysis data processor [E] providing a procedure for application to signals received from the second detector, the procedure using a value for each of one or more characteristics of the procedure, the procedure being provided for use when the signals received from the first analysis data processor [C] are of a given form, the second analysis data processor [E] providing a further procedure for application to the signals from the second detector, the further procedure using a different value for one or more of the characteristics, the further procedure being applied to the signals from the second detector to the second process controller when the signals differ from the given form, and
wherein the signals from the first analysis data processor [C] are provided to the second analysis data processor [E] after the second process controller has started applying the process to the second chamber.

## Patentansprüche

1. Verfahren zum Analysieren einer Probe [A], wobei das Verfahren Folgendes umfasst:
Bereitstellen eines ersten Teils der Probe;
Bereitstellen eines zweiten Teils der Probe;
wobei der erste Teil der Probe und der zweite Teil der Probe voneinander getrennt sind;
Durchführen einer ersten Analyse [B] am ersten Teil der Probe mit einem ersten Analysedatenprozessor [C] ;
Prüfen der Ergebnisse der ersten Analyse [B];
Durchführen einer zweiten Analyse [D, E] am zweiten Teil der Probe, wobei es sich bei der ersten Analyse [B] und der zweiten Analyse [D, E] um verschiedene Analyseverfahren handelt, die zweite Analyse [D, E] gemäß einem Verfahren oder einem weiteren Verfahren durchgeführt wird, das Verfahren einen Wert für jede von einer oder mehreren Eigenschaften des Verfahrens verwendet, das weitere Verfahren einen anderen Wert für ein oder mehrere Eigenschaften verwendet;
wobei die Prüfung [F, G] der Ergebnisse der ersten Analyse [B] verwendet wird, um zu bestimmen, ob das Verfahren oder ein weiteres Verfahren verwendet wird, das Verfahren dann zur Verwendung bereitgestellt wird, wenn die vom ersten Analysedatenprozessor empfangenen Signale eine bestimmte Form haben, das weitere Verfahren dann zur Verwendung bereitgestellt wird, wenn die vom ersten Analysedatenprozessor empfangenen Signale von der bestimmten Form abweichen; und
wobei die zweite Analyse [D, E] gestartet wird, bevor die Ergebnisse der ersten Analyse [B] erhalten werden.

2. Verfahren nach Anspruch 1, wobei die erste Analyse [B] und die zweite Analyse [D, E] innerhalb von 5 Minuten des Starts der jeweils anderen Analyse beginnen.

3. Verfahren nach Anspruch 1 oder Anspruch 2, wobei die erste Analyse [B] bis zum Abschluss weniger als 60 Minuten in Anspruch nimmt.

4. Verfahren nach einem vorhergehenden Anspruch, wobei die erste Analyse [B] mindestens 30 Minuten vor der geplanten Beendigung der zweiten Analyse [D, E] abgeschlossen ist.

5. Verfahren nach einem vorhergehenden Anspruch, wobei der erste Teil und der zweite Teil das gleiche Volumen ± 1% aufweisen.

6. Verfahren nach einem vorhergehenden Anspruch, wobei das Verhältnis von Probe zu Reagenzien in Bezug auf den ersten Teil und in Bezug auf den zweiten Teil gleich ist ± 1%.

7. Verfahren nach einem vorhergehenden Anspruch, wobei die erste Analyse [B] Information [C] in Bezug auf eine oder mehrere Eigenschaften des ersten Teils der Probe bereitstellt, wobei die eine oder mehrere Eigenschaften eine oder mehrere der Folgenden sind:
die Menge an Nukleinsäure;
die Menge an amplifizierbarer Nukleinsäure;
die Menge an amplifizierbarer Nukleinsäure eines bestimmten Typs;
die Anwesenheit eines oder mehrerer Amplifikationsinhibitoren;
das Ausmaß des Nukleinsäureabbaus;
das Vorhandensein von Nukleinsäure von Chromosom Y;
die Menge der Nukleinsäure von Chromosom Y;
die Anwesenheit von Nukleinsäure aus zwei oder mehreren verschiedenen Quellen;
das Verhältnis der Nukleinsäure aus einer Quelle zu der Nukleinsäure aus einer anderen Quelle.

8. Verfahren nach einem vorhergehenden Anspruch, wobei die zweite Analyse [D, E] gemäß einem Verfahren durchgeführt wird, das eine Standardform hat, und die Standardform des Verfahrens in der zweiten Analyse [D, E] verwendet wird, sofern nicht die Prüfung [F, G] der Ergebnisse aus der ersten Analyse [B] eine Veränderung des Verfahrens nahelegt.

9. Verfahren nach einem vorhergehenden Anspruch, wobei das Verfahren in Bezug auf eine oder mehrere Eigenschaften definiert ist, und der Wert für eine Eigenschaft eine Anzahl von PCR-Zyklen oder eine Arbeitstemperatur eines PCR-Zyklus oder eine Zeitdauer für einen PCR-Zyklus ist.

10. Verfahren nach einem vorhergehenden Anspruch, wobei der Wert für eine oder mehrere der Eigenschaften des Verfahrens in Reaktion auf die erste Analyse [B] geändert wird, was das Vorhandensein von einem oder mehreren Inhibitoren in der Probe [A] anzeigt.

11. Verfahren nach einem vorhergehenden Anspruch, wobei die Nukleinsäuremenge in der ersten Analyse [B] erfasst wird und mit einer Referenzmenge oder einem Referenzmengenbereich verglichen wird, die Referenzmenge oder der Referenzmengenbereich zur Verwendung in der zweiten Analyse [D, E] einem oder mehreren Werten zugeordnet wird, und wo die in der ersten Analyse [B] erfasste Menge unter der Referenzmenge oder dem Referenzmengenbereich ist, die Anzahl der Zyklen verglichen mit dem Wert der Zyklen, der den Referenzen zugeordnet wird, erhöht wird, und/oder wo die in der ersten Analyse [B] erfasste Menge über der Referenzmenge oder dem Referenzmengenbereich ist, die Anzahl der Zyklen verglichen mit dem Wert der Zyklen, der den Referenzen zugeordnet wird, verringert wird.

12. Verfahren nach einem vorhergehenden Anspruch, wobei die Prüfung der Ergebnisse der ersten Analyse [B] verwendet wird, um zu bestimmen, ob der Wert für eine oder mehrere der Eigenschaften des Verfahrens auf einen anderen Wert geändert wird, und der Wert in Bezug auf einen Wert ist, der in der zweiten Analyse [D, E] bei der physikalischen Verarbeitung [D] des zweiten Teils der Probe oder in der zweiten Analyse [D, E] in der zweiten Analysendatenverarbeitung [E] der Ergebnisse für den zweiten Teil der Probe verwendet wird.

13. Verfahren nach einem vorhergehenden Anspruch, wobei die Ergebnisse der ersten Analyse [B] anzeigen, ob die Probe aus einem Gemisch von Quellen stammt oder nicht, und/oder das Verhältnis von Nukleinsäure aus einer Quelle zu der aus einer anderen Quelle in dem Gemisch anzeigen.

14. Verfahren zum Analysieren einer Probe, wobei das Verfahren umfasst: die parallele Verarbeitung eines ersten Analyseverfahrens [B] und eines zweiten Analyseverfahrens [D, E], wobei die Ergebnisse des ersten Analyseverfahrens [B] geprüft werden [F, G] und verwendet werden, um das zweite Analyseverfahren [D, E] zu verbessern, bevor es abgeschlossen wird.

15. Vorrichtung zum Analysieren einer Probe [A], wobei die Vorrichtung Folgendes aufweist:
eine Probeneinleitungsstelle;
eine erste Kammer in Fluidverbindung mit der Probeneinleitungsstelle;
eine Verfahrenssteuervorrichtung für die erste Kammer;
einen ersten Detektor für eine oder mehrere Eigenschaften eines ersten Teils einer in der ersten Kammer gemäß der Verfahrenssteuervorrichtung für die erste Kammer verarbeiteten Probe;
einen ersten Analysedatenprozessor [C], der Signale von dem ersten Detektor empfängt und die Signale an eine zweite Verfahrenssteuervorrichtung weitergibt;
eine zweite Kammer in Fluidverbindung mit der Probeneinleitungsstelle, wobei die zweite Kammer einen zweiten Teil der Probe gemäß der zweiten Verfahrenssteuervorrichtung verarbeitet;
einen zweiten Detektor für eine oder mehrere Eigenschaften des zweiten Teils der in der zweiten Kammer verarbeiteten Probe;
wobei der erste Teil der Probe in der ersten Kammer und der zweite Teil der Probe in der zweiten Kammer voneinander getrennt sind;
wobei die erste Analyse [B] und die zweite Analyse [D, E] unterschiedliche Analyseverfahren sind;
wobei die zweite Verfahrenssteuervorrichtung ein Verfahren zur Anwendung auf die zweite Kammer bereitstellt, das Verfahren einen Wert für jede von einer oder mehreren Eigenschaften des Verfahrens verwendet, das Verfahren dann zur Verwendung bereitgestellt wird, wenn die vom ersten Analysedatenprozessor [C] empfangenen Signale eine bestimmte Form haben, die zweite Verfahrenssteuerungsvorrichtung ein weiteres Verfahren zur Anwendung auf die zweite Kammer bereitstellt, das weitere Verfahren einen unterschiedlichen Wert für eine oder mehrere der Eigenschaften verwendet, das weitere Verfahren auf die zweite Kammer in Reaktion auf die vom ersten Datenprozessor [C] an die zweite Verfahrenssteuerung weitergeleiteten Signale angewendet wird, wenn die Signale von der bestimmten Form abweichen; und
wobei die Signale vom ersten Analysedatenprozessor [C] an die zweite Verfahrenssteuervorrichtung weitergegeben werden, nachdem die zweite Verfahrenssteuervorrichtung das Anwenden des Verfahren auf die zweite Kammer begonnen hat.

16. Vorrichtung zum Analysieren einer Probe [A], wobei die Vorrichtung Folgendes aufweist:
eine Probeneinleitungsstelle;
eine erste Kammer in Fluidverbindung mit der Probeneinleitungsstelle;
eine Verfahrenssteuervorrichtung für die erste Kammer;
einen ersten Detektor für eine oder mehrere Eigenschaften eines ersten Teils einer in der ersten Kammer gemäß der Verfahrenssteuervorrichtung für die erste Kammer verarbeiteten Probe;
einen ersten Analysedatenprozessor [C], der Signale von dem ersten Detektor empfängt und die Signale an einen zweiten Analysedatenprozessor weitergibt;
eine zweite Kammer in Fluidverbindung mit der Probeneinleitungsstelle;
eine Verfahrenssteuervorrichtung für die zweite Kammer;
einen zweiten Detektor für eine oder mehrere Eigenschaften des zweiten Teils der in der zweiten Kammer gemäß der Verfahrenssteuervorrichtung für die zweite Kammer verarbeiteten Probe;
wobei der erste Teil der Probe in der ersten Kammer und der zweite Teil der Probe in der zweiten Kammer voneinander getrennt sind;
wobei die erste Analyse [B] und die zweite Analyse [D, E] unterschiedliche Analyseverfahren sind;
wobei der zweite Analysedatenprozessor [E] ein Verfahren zur Anwendung auf die von dem zweiten Detektor empfangenen Signale bereitstellt, das Verfahren einen Wert für jede von einer oder mehreren Eigenschaften des Verfahrens verwendet, das Verfahren dann zur Verwendung bereitgestellt wird, wenn die vom ersten Analysedatenprozessor [C] empfangenen Signale eine bestimmte Form haben, der zweite Analysedatenprozessor [E] ein weiteres Verfahren zur Anwendung auf die Signale von dem zweiten Detektor bereitstellt, das weitere Verfahren einen anderen Wert für eine oder mehrere der Eigenschaften verwendet, das weitere Verfahren auf die Signale von dem zweiten Detektor zur zweiten Verfahrenssteuervorrichtung angewendet wird, wenn die Signale von der bestimmten Form abweichen; und
wobei die Signale vom ersten Analysedatenprozessor [C] an den zweiten Analysedatenprozessor [E] weitergegeben werden, nachdem die zweite Verfahrenssteuervorrichtung das Anwenden des Verfahrens auf die zweite Kammer begonnen hat.

## Revendications

1. Procédé d'analyse d'un échantillon [A], le procédé comprenant :
la fourniture d'une première partie de l'échantillon ;
la fourniture d'une seconde partie de l'échantillon ;
la première partie de l'échantillon et la seconde partie de l'échantillon étant séparées l'une de l'autre ;
la mise en oeuvre d'une première analyse [B] sur la première partie de l'échantillon à l'aide d'un premier dispositif de traitement de données d'analyse [C] ;
la considération des résultats de la première analyse [B] ;
la mise en oeuvre d'une seconde analyse [D, E] sur la seconde partie de l'échantillon, la première analyse [B] et la seconde analyse [D, E] étant des processus d'analyse différents, la seconde analyse [D, E] étant mise en oeuvre selon une procédure ou une autre procédure, la procédure utilisant une valeur pour chacune d'une ou plusieurs caractéristiques de la procédure, l'autre procédure utilisant une valeur différente pour une ou plusieurs des caractéristiques ;
dans lequel la considération [F, G] des résultats de la première analyse [B] est utilisée pour déterminer si la procédure ou une autre procédure est utilisée, la procédure étant destinée à être utilisée lorsque les signaux reçus à partir du premier dispositif de traitement de données d'analyse sont de forme donnée, l'autre procédure étant destinée à être utilisée lorsque les signaux reçus à partir du premier dispositif de traitement de données d'analyse diffèrent de la forme donnée ; et
dans lequel la seconde analyse [D, E] est commencée avant que les résultats de la première analyse [B] soient obtenus.

2. Procédé selon la revendication 1, dans lequel la première analyse [B] et la seconde analyse [D, E] commencent à moins de 5 minutes de l'autre début d'analyse.

3. Procédé selon la revendication 1 ou la revendication 2, dans lequel la première analyse [B] prend moins de 60 minutes pour être terminée.

4. Procédé selon une quelconque revendication précédente, dans lequel la première analyse [B] est terminée au moins 30 minutes avant la fin programmée de la seconde analyse [D, E].

5. Procédé selon une quelconque revendication précédente, dans lequel la première partie et la seconde partie ont le même volume ± 1 %.

6. Procédé selon une quelconque revendication précédente, dans lequel le rapport de l'échantillon aux réactifs est le même en ce qui concerne la première partie et en ce qui concerne la seconde partie ± 1 %.

7. Procédé selon une quelconque revendication précédente, dans lequel la première analyse [B] fournit des informations [C] sur une ou plusieurs caractéristiques de la première partie de l'échantillon, la ou les caractéristiques étant une ou plusieurs caractéristiques parmi :
la quantité d'acide nucléique ;
la quantité d'acide nucléique amplifiable ;
la quantité d'acide nucléique amplifiable d'un type donné ;
la présence d'un ou plusieurs inhibiteurs vis-à-vis de l'amplification ;
le degré de dégradation de l'acide nucléique ;
la présence d'acide nucléique du chromosome Y ;
la quantité d'acide nucléique du chromosome Y ;
la présence d'acide nucléique provenant de deux ou plus de deux sources différentes ;
le rapport de l'acide nucléique provenant d'une source à l'acide nucléique provenant d'une autre source.

8. Procédé selon une quelconque revendication précédente, dans lequel la seconde analyse [D, E] est mise en oeuvre selon une procédure qui a une forme standard et la forme standard de la procédure est utilisée dans la seconde analyse [D, E] sauf si la considération [F, G] des résultats provenant de la première analyse [B] suggèrent un changement à apporter à la procédure.

9. Procédé selon une quelconque revendication précédente, dans lequel la procédure est définie en termes d'une ou plusieurs caractéristiques et la valeur pour une caractéristique est un nombre de cycles de PCR ou une température de fonctionnement de cycle de PCR ou une durée pour un cycle de PCR.

10. Procédé selon une quelconque revendication précédente, dans lequel la valeur pour une ou plusieurs des caractéristiques de la procédure est changée en réponse à la première analyse [B] indiquant la présence d'un ou plusieurs inhibiteurs dans l'échantillon [A].

11. Procédé selon une quelconque revendication précédente, dans lequel la quantité d'acide nucléique est détectée dans la première analyse [B] et est comparée à une quantité de référence ou une plage de quantités de référence, la quantité ou plage de quantités de référence étant associée à une ou plusieurs valeurs à utiliser dans la seconde analyse [D, E], quand la quantité détectée dans la première analyse [B] est au-dessous de la quantité de référence ou de la plage de quantités de référence, le nombre de cycles étant accru par comparaison avec la valeur de cycles associée aux références et/ou quand la quantité détectée dans la première analyse [B] est au-dessus de la quantité de référence ou de la plage de quantités de référence, le nombre de cycles étant diminué par comparaison avec la valeur de cycles associée aux références.

12. Procédé selon une quelconque revendication précédente, dans lequel la considération des résultats de la première analyse [B] est utilisée pour déterminer si la valeur pour une ou plusieurs des caractéristiques de la procédure est changée en une valeur différente et la valeur concerne une valeur utilisée dans la seconde analyse [D, E] dans le traitement physique [D] de la seconde partie de l'échantillon ou dans la seconde analyse [D, E] dans le second traitement de données d'analyse [E] des résultats pour la seconde partie de l'échantillon.

13. Procédé selon une quelconque revendication précédente, dans lequel les résultats de la première analyse [B] indiquent si l'échantillon provient ou non d'un mélange de sources et/ou le rapport d'acide nucléique provenant d'une source à celui provenant d'une autre source dans le mélange.

14. Procédé d'analyse d'un échantillon, le procédé comprenant : la mise en oeuvre en parallèle d'un premier procédé d'analyse [B] et d'un second procédé d'analyse [D, E], les résultats du premier procédé d'analyse [B] étant considérés [F, G] et utilisés pour modifier le second procédé d'analyse [D, E] avant qu'il soit terminé.

15. Appareil pour l'analyse d'un échantillon [A], l'appareil comprenait :
un lieu d'introduction d'échantillon ;
une première chambre en communication fluidique avec le lieu d'introduction d'échantillon ;
un dispositif de commande de processus dans la première chambre ;
un premier détecteur pour une ou plusieurs propriétés d'une première partie d'un échantillon traitée dans la première chambre selon le dispositif de commande de processus dans la première chambre ;
un premier dispositif de traitement de données d'analyse [C] recevant des signaux provenant du premier détecteur et fournissant des signaux à un second dispositif de commande de processus ;
une seconde chambre en communication fluidique avec le lieu d'introduction d'échantillon, la seconde chambre traitant une seconde partie de l'échantillon selon le second dispositif de commande de processus ;
un second détecteur pour une ou plusieurs propriétés de la seconde partie de l'échantillon traitée dans la seconde chambre ;
la première partie de l'échantillon dans la première chambre et la seconde partie de l'échantillon dans la seconde chambre étant séparées l'une de l'autre ;
la première analyse [B] et la seconde analyse [D, E] étant des processus d'analyse différents ;
le second dispositif de commande de processus fournissant une procédure destinée à être appliquée à la seconde chambre, la procédure utilisant une valeur pour chacune d'une ou plusieurs caractéristiques de la procédure, la procédure étant destinée à être utilisée lorsque les signaux reçus à partir du premier dispositif de traitement de données d'analyse [C] sont de forme donnée, le second dispositif de commande de processus fournissant une autre procédure destinée à être appliquée à la seconde chambre, l'autre procédure utilisant une valeur différente pour une ou plusieurs des caractéristiques, l'autre procédure étant appliquée à la seconde chambre en réponse aux signaux fournis par le premier dispositif de traitement de données [C] au second dispositif de commande de processus lorsque les signaux diffèrent de la forme donnée et
dans lequel les signaux provenant du premier dispositif de traitement de données d'analyse [C] sont fournis au second dispositif de commande de processus après que le second dispositif de commande de processus a commencé à appliquer la procédure à la seconde chambre.

16. Appareil pour l'analyse d'un échantillon [A], l'appareil comprenant :
un lieu d'introduction d'échantillon ;
une première chambre en communication fluidique avec le lieu d'introduction d'échantillon ;
un dispositif de commande de processus dans la première chambre ;
un premier détecteur pour une ou plusieurs propriétés d'une première partie d'un échantillon traitée dans la première chambre selon le dispositif de commande de processus dans la première chambre ;
un premier dispositif de traitement de données d'analyse [C] recevant des signaux provenant du premier détecteur et fournissant des signaux à un second dispositif de traitement de données d'analyse ;
une seconde chambre en communication fluidique avec le lieu d'introduction d'échantillon ;
un dispositif de commande de processus dans la seconde chambre ;
un second détecteur pour une ou plusieurs propriétés de la seconde partie de l'échantillon traitée dans la seconde chambre selon le dispositif de commande de processus dans la seconde chambre ;
la première partie de l'échantillon dans la première chambre et la seconde partie de l'échantillon dans la seconde chambre étant séparées l'une de l'autre ;
la première analyse [B] et la seconde analyse [D, E] étant des processus d'analyse différents ;
le second dispositif de traitement de données d'analyse [E] fournissant une procédure destinée à être appliquée à des signaux reçus à partir du second détecteur, la procédure utilisant une valeur pour chacune d'une ou plusieurs caractéristiques de la procédure, la procédure étant destinée à être utilisée lorsque les signaux reçus à partir du premier dispositif de traitement de données d'analyse [C] sont de forme donnée, le second dispositif de traitement de données d'analyse [E] fournissant une autre procédure destinée à être appliquée au signaux provenant du second détecteur, l'autre procédure utilisant une valeur différente pour une ou plusieurs des caractéristiques, l'autre procédure étant appliquée aux signaux provenant du second détecteur et allant au second dispositif de commande de processus lorsque les signaux diffèrent de la forme donnée et
dans lequel les signaux provenant du premier dispositif de traitement de données d'analyse [C] sont fournis au second dispositif de traitement de données d'analyse [E] après que le second dispositif de commande de processus a commencé à appliquer le processus à la seconde chambre.
